89117475.7

**ELISABETH JUNG** DR. PHIL., DIPL.-CHEM.
**JÜRGEN SCHIRDEWAHN** DR. RER. NAT., DIPL.-PHYS.
**CLAUS GERNHARDT** DIPL.-ING.

PATENTANWÄLTE
EUROPEAN PATENT ATTORNEYS

8000 MÜNCHEN 40,
P. O. BOX 40 14 68
CLEMENSSTRASSE 30
TELEFON: (089) 34 50 67
TELEGRAMM/CABLE: INVENT MÜNCHEN
TELEX: 5-29 686
TELECOPIERER (FAX): (089) 39 92 39 (GR. II/III)

S 792 C/I

(J/sei)

September 21, 1989

DIVISIONAL APPLICATION

from EP 84902363.5-2110/JP8400305

WAKO PURE CHEMICAL INDUSTRIES, LTD.

10, Doshomachi-3-chome

Higashi-ku, Osaka, Japan

---

METHOD OF QUANTITATIVELY MEASURING AN OXIDATIVE SUBSTANCE BY USING TRIPHENYL METHANE TYPE LEUCO COMPOUNDS AS COLORING MATTER

---

Priority Dates:

Japan - March 15,1984, No. 49950/84

Japan - April 2, 1984, No. 65629/84

Japan - April 10,1984, No. 71548/84

(19) Europäisches Patentamt
European Patent Office
Office européen des brevets

(11) Publication number: 0 355 864 A2

(12) **EUROPEAN PATENT APPLICATION**

(21) Application number: 89117475.7

(22) Date of filing: 12.06.84

(51) Int. Cl.4: C09B 11/12, C12Q 1/28, G01N 33/50

The application is published incomplete as filed (Article 93 (2) EPC). The point in the claims at which the omission obviously occurs has been left blank.

A request for addition of a missing page of the claims has been filed pursuant to Rule 88 EPC. A decision on the request will be taken during the proceedings before the Examining Division (Guidelines for Examination in the EPO, A-V, 2.2).

(30) Priority: 15.03.84 JP 49950/84
02.04.84 JP 65629/84
10.04.84 JP 71548/84

(43) Date of publication of application: 28.02.90 Bulletin 90/09

(60) Publication number of the earlier application in accordance with Art.76 EPC: 0 174 371

(84) Designated Contracting States: AT BE CH DE FR GB LI LU NL SE

(71) Applicant: WAKO PURE CHEMICAL INDUSTRIES, LTD.
10, Doshomachi-3-chome
Higashi-ku Osaka(JP)

(72) Inventor: Kazuhiko, Yamanashi
17-10, Akatuka 3-chome Itabashi-Ku
Tokyo 175(JP)
Inventor: Toshiro, Hanada
103 Minami Haitum 784 Oaza Minamiotuka
Kawagoe-shi Saitama 350(JP)

(74) Representative: Dr. Elisabeth Jung Dr. Jürgen Schirdewahn Dipl.-Ing. Claus Gernhardt
P.O. Box 40 14 68 Clemensstrasse 30
D-8000 München 40(DE)

(54) **Method of quantitatively measuring an oxidative substance by using triphenyl methane type leuco compounds as coloring matter.**

(57) The invention is to provide a quantitatively measuring method of an oxidative substance with the influences of coloring-interfering substances being avoided and enabling the adjustment of the coloring sensitivity. The coloring reagent used is represented by the following formula (I):

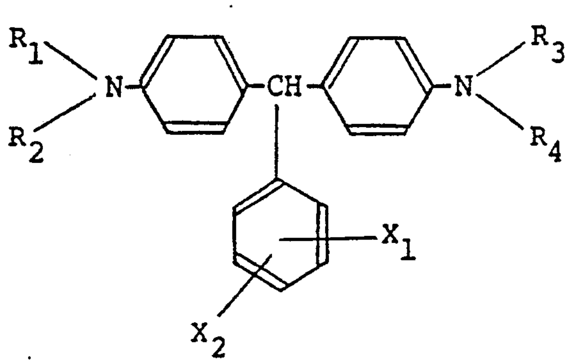

(I)

wherein $R_1$, $R_2$, $R_3$ and $R_4$, which may be the same or different from one another, represent a hydrogen atom or a $C_{1-3}$-alkyl group, and $X_1$ and $X_2$, which may be the same as or different from each other,

EP 0 355 864 A2

represent a hydrogen atom, $-SO_3M_1$, $-COOM_2$, $-O(CH_2)_mSO_3M_3$, $-O(CH_2)_nCOOM_4$ or $-N(R_5)(R_6)$ in which $M_1$, $M_2$, $M_3$ and $M_4$ represent a hydrogen atom, an alkali metal ion or $NH_4^+$, $R_5$ and $R_6$ independently represent a hydrogen atom or a lower alkyl group, and m and n represent an integer of 2 - 4.

According to one embodiment at least one kind of (i) uricase, (ii) an anionic surface active agent and (iii) a metal chelate compound is added, whereby influences of a coloring-interfering substance is avoided.

According to another embodiment an azide is used as sensitivity adjustor or cyclodextrin or a modified cyclodextrin is included for the same purpose.

FIG. 1

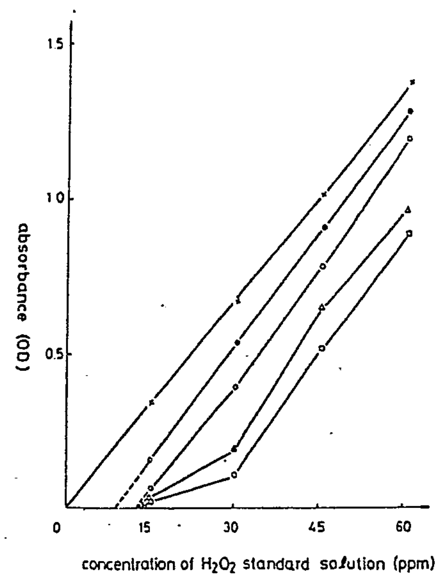

# METHOD OF QUANTITATIVELY MEASURING AN OXIDATIVE SUBSTANCE BY USING TRIPHENYL METHANE TYPE LEUCO COMPOUNDS AS COLORING MATTER

## TECHNICAL FIELD

The present invention relates to a method of quantitatively measuring an oxidative substance by using a triphenyl methane type leuco coloring matter in which the influences of a coloring-interfering substance are avoided as well as a method of quantitatively measuring an oxidative substance which enables the adjustment of the coloring sensitivity.

## BACKGROUND TECHNIQUE

Since changes in components of body fluids such as blood, urine and the like are closely correlated with diseases, the measurement of the body fluid components has become important in order to diagnose the diseases, to solve pathological conditions and to select a therapeutic course. For instance, it is well known that there have been developed methods for measuring a great variety of micro-components including cholesterol, triglyceride, glucose, uric acid, monoamine oxidase, bile acid and the like in the blood, and they have been used for the diagnosis of the diseases.

At present, as a method of measuring serum components there is generally popularized a so-called "enzyme method" in which an enzyme reaction is carried out by using an enzyme specifically acting upon a target component, and a product formed thereby is measured to determine the content of the target component. With the development of oxidizable coloring reagents, there has been developped and used, for example, a method in which an oxidizable coloring reagent is oxidized with a peroxidase-like substance and an oxidative substance to form a coloring system. Representative for this technique is a method in which an $H_2O_2$ generating enzyme, for instance, an oxidase, is used to generate $H_2O_2$ in an amount necessary for the respective target component and the thus generated $H_2O_2$ is forming a coloring system with a peroxidase and an oxidizable coloring reagent as a coloring component, the amount of the target component being determined through colorimetric determination. By way of example there may be recited a method in which $H_2O_2$ is generated by the combined use of cholesterol-cholesterol oxidase, glucose-glucose oxidase, triglyceride-lipoprotein lipase-glycerol oxidase, uric acid-uricase, or the like, forming a coloring system by using peroxidase and an oxidizable coloring reagent, the color development being measured by using a photometer to determine the amount of a target component. As a matter of course, the concentrations of the components in the serum differ depending upon the kinds of the components, and therefore the amount of $H_2O_2$ generated through the enzyme reaction varies over a wide range. Accordingly, there have been developed and used oxidizable coloring reagents with sensitivities meeting the intended purposes. For example, oxidizable coloring reagents combining 4-aminoantipyrine with a phenolic compound of a N,N-dialkylaniline compound are generally used in the measurement of cholesterol, triglyceride, glucose, uric acid or the like.

However, among the body fluid components, there are some components which are contained in the normal serum in an extremely small amount, like monoamine oxidase and bile acid. The monoamine oxidase is an enzyme which acts upon a monoamine compound to generate $H_2O_2$ and aldehyde, but the amount of the generated $H_2O_2$ is extremely small due to its extremely small concentration in the serum. Thus, the oxidizable coloring reagents of the above combinations are insufficient in sensitivity to quantitatively determine such components. Consequently, an oxidizable coloring reagent with a higher sensitivity is being demanded. Under these circumstances, there has heretofore been developed and used on a commercial base a measuring method using a derivative of leucomethylene blue as the oxidizable coloring reagent with a higher sensitivity. However, the leuco coloring matter has the defect that its stability in a solution is insufficient, and therefore a coloring reagent containing this leuco coloring matter is gradually colored during storage.

Furthermore, although for similar purposes there have been studied a leucocrystal violet, a leuco malachite green and so on as an oxidizable coloring reagent with high sensitivity, all of these are difficult to dissolve in water in the neutral pH-range. Therefore, it is difficult to dissolve them in a desired concentration. Thus, they are unsuitable for the measurement of a micro-component.

As a leuco coloring matter improved in its water solubility, there has been proposed bis(4-diethylaminophenyl)-2-sulfophenyl methane (hereinafter abbreviated as BSPM) (Japanese Application Laid-Open No. 26199/1981). However, it can not necessarily be said that the solubility thereof in water is sufficient.

Another technical problem is practising such a method of quantitatively measuring an oxidative substance consists in that body fluids rather often comprise a coloring-interfering substance, like uric acid and protein, thereby endangering the accuracy of the measurement.

It is therefore an object of the present invention to provide a method of quantitatively measuring an oxidative substance in which the influences of a coloring-interfering substance are avoided.

It is still another object of the present invention to provide a method of quantitatively measuring an oxidative substance in which the coloring sensitivity is adjusted for practical purposes so that an ordinary photometer with a logarithm scale may be used.

Surprisingly the present inventors have found in practising the invention that the influence of uric acid can be avoided when uricase is coexistent (in this case, the uric acid can be decomposed without being accompanied by the production of $H_2O_2$), while the influence of protein can be avoided through the addition of a metal chelate or a specific surface active agent. By those measures the influences of the color-interfering substance can be avoided.

Therefore, according to the present invention the leuco coloring matter can be used for the measurement of chemical components in body fluids such as the serum or the blood containing uric acid or protein according to the enzyme method ($H_2O_2$ generating system) without any trouble.

As the chemical components in body fluids which can be measured by the method of the invention, mention may be made of all target compounds which are measured according to the conventional enzyme method($H_2O_2$ generating system), for instance, glucose, free cholesterol, whole cholesterol, HDL (high specific density lipoprotein)-cholesterol, LDL (low specific density lipoprotein)-cholesterol, triglyceride, phospholipid, uric acid, monoamine oxidase and so on (in the case of uric acid it is necessary that uricase is preliminarily acted upon uric acid to generate $H_2O_2$, and then the coloring reagent according to the present invention is added together with peroxidase for color development).

Therefore according to the invention the method of quantitatively measuring an oxidative substance by using as a coloring reagent a triphenyl methane type leuco coloring matter represented by the general formula (I):

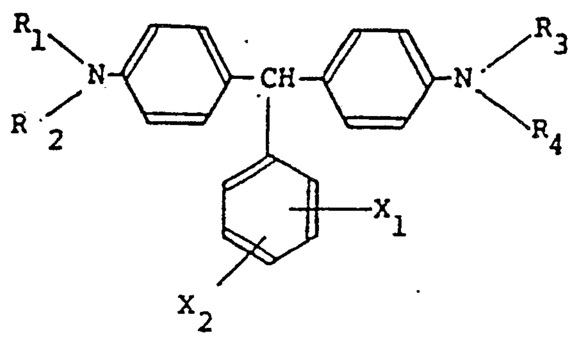

(I)

wherein $R_1$, $R_2$, $R_3$ and $R_4$, which may be the same or different from one another, represent a hydrogen atom or a $C_{1-3}$-alkyl group, and $X_1$ and $X_2$, which may be the same as or different from each other, represent a hydrogen atom, $-SO_3M_1$, $-COOM_2$, $-O(CH_2)_mSO_3M_3$, $-O(CH_2)_nCOOM_4$ or $-N(R_5)(R_6)$ in which $M_1$, $M_2$, $M_3$ and $M_4$ represent a hydrogen atom, an alkali metal ion or $NH_4^+$ , $R_5$ and $R_6$ independently represent a hydrogen atom or a lower alkyl group, and m and n represent an integer of 2 - 4, is characterized by using at least one kind of (i) uricase, (ii) an anionic surface active agent and (iii) a metal chelate compound, whereby influences of a coloring-interfering substance is avoided.

In practising the method of the invention with respect to the serum, a concentration of more than 0.01 mM of the triphenyl methane type oxidizable coloring reagent is suitable, and in general a concentration of 0.02 - 0.3 mM is preferably used.

As specific examples of triphenyl methane leuco type coloring matter suitable for the method of the invention there may be mentioned leuco crystal violet, leucomalachite green and the above-mentioned compound BSPM.

Most suitable coloring matter are also the novel compounds described and claimed in the parent

application No.84902363.5 which are characterized in that $R_1$, $R_2$, $R_3$ and $R_4$ in formula (I) are the same or different from one another and represent a $C_{1-3}$-alkyl group, whereas $X_1$ and $X_2$, which may be the same or different from each other represent a group $-O(CH_2)_mSO_3M_3$ ("m" and "$M_3$" having the meaning as stated above) or either of $X_1$ and $X_2$ represents a group $-O(CH_2)_mSO_3M_3$ and the other represents a hydrogen atom.

As the surface active agents effectively used in the present invention for avoiding the interference of protein, generally, anionic surface active agents, particularly the ones represented by the following formulae (II) and (III), are effective.

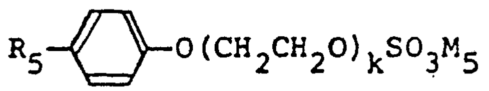 (II)

$$R_6O(CH_2CH_2O)_{\ell}SO_3M_6 \quad \text{(III)}$$

wherein $R_5$ represents an alkyl group of 8 - 9 carbon atoms, $R_6$ represents an alkyl group of 8 - 18 carbon atoms, $M_5$ and $M_6$ each represent an alkali metal ion, an ammonium ion, or a quaternary ammonium ion, and k and $\ell$ each represent an integer of 1 - 6. As specific examples corresponding to the compound of the general formula (II), mention may be made of Emal® NC(polyoxyethylene alkylphenyl ether sodium sulfate; trademark of Kao Soap Co., Ltd.), Newcol® 560 SF (polyoxyethylene nonylphenyl ether ammonium sulfate; trademark of Nippon Emulsifier Co., Ltd.),Newcol® SNP-4T (polyoxyethylene nonylphenyl ether triethanolamine sulfate; trademark of Nippon Emulsifier Co., Ltd.). As specific examples corresponding to the compound of the general formula (III), mention may be made of Emal® 20 C (polyoxyethylene alkyl ether sodium sulfate; trademark of Kao Soap Co., Ltd.), Sannol® 605 D (polyoxyethylene alkyl ether sodium sulfate; trademark of Lion Co., Ltd.), Nicol® NES-303 (polyoxyethylene alkyl ether triethanolamine sulfate; trademark of Nikko Chemicals Co., Ltd.). As a matter of course, the invention is not limited to those compounds specifically mentioned above. These surface active agents are each used in general at the actual concentration of 0.01 - 10% in the coloring reagent solution.

As the metal chelate compounds effective for removing the interference of protein, generally a metal-EDTA (ethylenediamine tetraacetic acid) is effectively used. As specific examples of the chelate, mention may be made of, for instance, Fe(III)-EDTA, Mn(II)-EDTA, Ni(II)-EDTA and the like, but they are not restrictive. All the ordinary metal-EDTA chelates commercially available may be used. An effect can already be observed by adding an amount of not less than 0.005%, and ordinarily 0.01 - 0.5% is preferably adopted.

However, uricase is used for avoiding the interference of uric acid being an oxidation enzyme for uric acid and acting upon uric acid which is converted into allantoin, hydrogen peroxide and carbon dioxide. According to the present invention, uric acid can be decomposed without being accompanied by the formation of $H_2O_2$ by acting uricase upon the uric acid in the presence of the triphenyl methane derivative and peroxidase. Thus, the triphenyl methane type leuco-pigment is not colored at all and uric acid as the interfering substance can be extremely easily and effectively removed. The addition amount of uricase may be generally not less than 50 U/$\ell$, and a range of 100 - 500 U/$\ell$ is preferably used. When a reagent solution containing 0.01 - 0.3 m mol/$\ell$ of the triphenyl methane type leuco-pigment, 50 - 500 U/$\ell$ of uricase, 100 - 10,000 U/$\ell$ of peroxidase, and an appropriate amount of a surface active agent, if necessary in a buffer solution of pH 6 - 8, is added to a sample containing uric acid, for instance, the uric acid is instantly decomposed. At that time, however, no hydrogen peroxide is generated and therefore, the triphenyl methane type leuco-pigment is not colored at all. It is presumed that the uric acid is decomposed to be converted into alloxan or a decomposition product thereof and urea. Anyway, there has been no literature describing the effect that uric acid can be decomposed by uricase without being accompanied by the generation of $H_2O_2$ . The present inventors first have found that while such a phenomenon is not recognized with respect to other oxidizable coloring reagent, it is an effect peculiar to the triphenyl leuco-pigment. This phenomenon could not be foreseen and is unexpected in that it involves an action utterly different from the ordinary enzyme action of uricase upon uric acid.

Since both protein and uric acid are ordinarily present in serum, when the component in the body fluid is measured according to the enzyme method ($H_2O_2$-POD system) or hemoglobin in the serum is measured according to the $H_2O_2$ system by using the triphenyl methane type leuco-pigment as a coloring reagent, the interferences of both the protein and the uric acid can be avoided by the combined use of the anionic surface active agent or metal chelate compound and uricase according to the present invention, so that

more precisely measured values can be easily obtained.

Although some of the coloring reagents used in the invention and represented by the general formula (I) are characterized by their high sensitivity, especially those described and claimed in parent application No. 84902363.5, the high sensitivity in turn becomes an obstacle in the practical use. Due to this high sensitivity a sample must be taken in an extremely small amount (for instance 10 $\mu l$ or less) in the measurement of the concentration of $H_2O_2$ in order that it can be satisfactorily measured with a sensitivity equivalent to that of a 4-aminoantipyrinphenol type coloring reagent. Therefore it is necessary to preliminarily dilute the sample in order to lessen the error incident to the sampling.

Such problems can be eliminated by another embodiment of the method according to the present invention by using an azide compound as sensitivity adjustor or by including cyclodextrin (hereinafter abbreviated as CD) or a modified cyclodextrin (hereinafter abbreviated as modified CD) in the quantitative measurement of the oxidative substance by using the triphenyl methane derivatives of the general formula (I) as a coloring reagent.

The azide compound, particularly sodium azide, is generally used as an antiseptic, and it is famous as an inhibitor against enzymes, such as catalase, peroxidase, lactase and the like.

However, in the present invention the effect is made use of which has not been heretofore known with respect to the conventional azide compound, that is, the effect of adjusting the coloring sensitivity of the triphenyl methane derivatives. This embodiment enables an application of the invention to a wide range of uses especially through utilization of the excellent characteristics of the novel triphenyl methane derivatives claimed and described in the parent application as the oxidizable coloring reagent, i.e. the coloring wave being on the long wavelength side and almost no color fading occurring with lapse of time after color development, thereby avoiding any restriction of the quantatitive measurement to an extremely small amount of substances.

As the azide, use may be made of any of alkali salt, alkaline earth metal salt, and other metal salt of hydrogen azide. Ordinarily, sodium azide is preferably used. So long as the concentration of the azide in the coloring reagent solution is not less than 0.001%, the effect for the reduction in the coloring sensitivity is recognized, and ordinarily 0.01 - 0.5 % is preferably used. For instance, in the case of BSdiproPM, the coloring sensitivity is lowered down to 16.5% at the maximum by adding 0.2% of sodium azide to the coloring reagent solution, and its calibration curve becomes a straight line passing through the origin. Moreover, according to the present invention, it is possible to appropriately adjust the coloring sensitivity to a desired range by appropriately selecting the addition amount of the azide.

Furthermore it is known that when CD or modified CD is mixed with an organic compound in an aqueous solution, they ordinarily readily form an inclusion body, and therefore, they have excellent effects, for example, in stabilizing medicines, adjusting the solubility, pulverizing liquid chemicals, masking stimulous and offensive odor and the like, adjusting the volatility, and they are accordingly used for such purposes. In addition, it is considered that when CD or modified CD is added to the coloring reagent, the coloring sensitivity is ordinarily increased by the effect of increasing the solubility, and therefore they are often used for this purpose, too.

However, according to the present invention, unexpectedly, the coloring sensitivity can be lowered by including the triphenyl methane derivatives with CD or modified CD, and consequently the derivatives can be applied over a wide range of uses exploiting their excellent characteristics without being restricted to the quantitative measurement of an extremely small amount of a substance.

In addition, since bis(p-diethylaminophenyl)-2-sulfophenyl methane (BSPM) (Japanese Patent Application Laid-Open No. 26,199/1981) which is one of the triphenyl methane derivatives of formula (I) and a known compound as a coloring reagent, is difficult to be completely dissolved even at the concentration of 0.01 mM in a buffer solution of pH 6 - 8 ordinarily adopted in the enzyme measurement method ($H_2O_2$ generation system) of an extremely small amount of a component in serum (ordinarily, it is dissolved in the acidic range, and then the pH-value is adjusted around neutrality, but the usable concentration is 0.5 mM at the maximum), it has been up to now difficult to put it into practical use in this field. To the contrary, according to the present invention, the solubility is increased by including CD or modified CD, so that the triphenyl methane derivatives can be dissolved even up to concentrations of 20 mM or more and thereby its practical use becomes possible.

CD used in the present invention can be applicable in any of its $\alpha$, $\beta$, $\gamma$, and $\delta$ forms. As the modified CD, mention may be made of, for instance, the compound of the following formula (IV):

$\beta\text{-CD(OH)}_{21}\text{-}m''(\text{-OZ}^1)_{m''}$ (IV)

wherein CD represents the cyclodextrin residue, $Z^1$ represents $-NO_2$, $-PO_3H$, $-SO_3H$ or a group of the formula: $-(CH_2)_{n''}Z^2$ in which $Z^2$ represents $-SO_3H$ or $-CH_2H$, and $n''$ is an integer of 1 to 4, and $m''$ represents an number of 1 to 5, and the compound of the general formula (V):

$\beta$-CD(-OH)$_{21}$-k′(-OCH$_3$)k′ (V)

wherein k′ represents a figure of higher than 0 but not higher than 21.

Typical examples of such $\beta$-cyclodextrin derivatives are enumerated by way of example as follows:

$\beta$-CD(-OH)$_{19}$(-ONO$_2$)$_2$

$\beta$-CD(-OH)$_{19.2}$(-OPO$_3$H)$_{1.8}$

$\beta$-CD(-OH)$_{19}$(-OSO$_3$H)$_2$

$\beta$-CD(-OH)$_{18.5}$(-O-CH$_2$-CO$_2$H)$_{2.5}$

$\beta$-CD(-OH)$_{19.3}$(-O-CH$_2$-CH$_2$-CH$_2$-SO$_3$H)$_{1.7}$

$\beta$-CD(-OH)$_{18.5}$(-O-CH$_2$-CH$_2$-CH$_2$-SO$_3$H)$_{2.5}$

$\beta$-CD(-OH)$_{18.0}$(-O-CH$_2$-CH$_2$-CH$_2$-SO$_3$H)$_{3.0}$

$\beta$-CD(-OH)$_7$(-OCH$_3$)$_{14}$

$\beta$-CD(-OCH$_3$)$_{21}$

However, the modified CD in the present invention is not restricted to these specific examples. These modified CD can be easily produced according to the ordinary producing processes described in the literatures. For instance, see Journal of Synthetic Organic Chemistry, Japan Vol. 35, No. 2, p 123 (41) to p 124 (42) (1977).

In order to include it with CD or modified CD, the triphenyl methane derivatives may be added and dissolved in a solution in which CD or the modified CD is dissolved in water or a buffer solution. The amount of CD or modified CD may be equal or more on a mole basis with respect to the triphenyl methane derivatives, and ordinarily, 10 - 1,000 times in mole is preferably adopted. By including the derivative with CD or modified CD, the coloring sensitivities are lowered to around 38.7% at most with respect to a $\alpha$-CD inclusion and around 29.0% at most with respect to $\beta$-CD inclusion in the case of BSdiproPM; around 53.7% at most with respect to $\alpha$-CD inclusion and around 38.8% at most with respect to $\beta$-CD inclusion in the case of BSproPM.

In the method of the present invention, the use of azide, CD or modified CD has no influence upon the coloring speed.

The method according to the present invention can be applied to the measuring of all target compounds which are measured by the conventional enzyme methods ($H_2O_2$ generation system). Measurement of a peroxidase-like substance using $H_2O_2$, for instance, is used for the measurement of substrates such as cholesterol, phospholipid, glucose, creatine, triglyceride, uric acid and so on in the body fluids, the measurement of the activity of the serum enzyme and the quantitative measurement of a hemoglobin in the serum (provided that in the case of uric acid $H_2O_2$ is produced in advance by acting uricase upon the uric acid, and the triphenyl methane derivative which is included with $\beta$-cyclodextrin is added together with peroxidase to develop the color.)

## BRIEF DESCRIPTION OF THE DRAWINGS

Fig.1 illustrates results in Table 1 regarding Example 2, in which absorbances (OD) are plotted on an ordinate and the respective concentration (ppm) of $H_2O_2$ standard solutions on the abscissa, and the plotted points are connected;

(-x-x-, -•-•-, -o-o-, -Δ-Δ-, and -□-□- show the calibration curves for the concentrations of the uric acid standard solutions, 0 mg/ℓ (water), 25 mg/ℓ, 50 mg/ℓ, 75 mg/ℓ, and 100 mg/ℓ respectively);

Fig. 2 shows a calibration curve obtained in Example 4 in which absorbances (OD) are plotted on an ordinate and the respective monoamine oxidase activities (IU/ℓ) on an abscissa, and the plotted points are connected

Fig.3 shows calibration curves obtained in Examples 5 and 6 in which absorbances (OD) are plotted on an ordinate, the respective concentrations (ppm) of hemoglobin (mg/ℓ) on an abscissa, and the plotted points are connected

(-x-x-, and -•-•- shows the calibration curves in Examples 5 and 6 respectively);

Fig. 4 shows calibration curves obtained in Examples 7 and 8 in which absorbances (OD) are plotted on an ordinate and the respective hydrogen peroxide concentrations (mg/ℓ) on an abscissa, and the plotted points are connected

(-x-x- and -o-o- show the calibration curves in Reference Examples 7 and 8 respectively);

Fig.5 shows calibration curves obtained in Examples 9 and 10 in which absorbances (OD) are plotted on an ordinate and the respective cholesterol concentrations (mg/dℓ)on an abscissa, and the plotted points

are connected
(-x-x-, and -o-o- show the calibration curves in Example 9 and 10 respectively);

Fig.6 shows calibration curves of $H_2O_2$ obtained in Reference Examples 11, 12 and 13 using BSPM as a coloring reagent in which -x-x-, -•-•- and -o-o- show the calibration curves in Examples 11, 12 and 13 respectively;

Fig.7 shows calibration curves of $H_2O_2$ in Reference Examples 14, 15 and 16 using BSdiproPM as a coloring reagent in which -x-x-, -•-•- and -o-o- show the calibration curves in Reference Examples 14, 15 and 16 respectively;

Fig.8 shows calibration curves of $H_2O_2$ in Reference Examples 17, 18 and 19 using BSproPM as a coloring reagent in which -x-x-, -•-•- and -o-o- show the calibration curves in Reference Examples 17, 18 and 19 respectively;

Fig.9 shows calibration curves for quantitatively measuring the whole cholesterol content in Examples 20 and 21 by using BSdiproPM as a coloring reagent in which -x-x- and -o-o- show the calibration curves in Examples 20 and 21 respectively;

Fig. 10 shows calibration curves for quantitatively measuring the whole cholesterol content in Examples 21 and 22 by using BSdiproPM as a coloring reagent in which -x-x- and -o-o- show the calibration curves in Examples 21 and 22 respectively; and

Fig.11 shows calibration curves for quantitatively measuring the triglyceride content in Examples 23 and 24 by using BSdiproPM as a coloring reagent in which -x-x- and -o-o- show the calibration curves in Examples 23 and 24 respectively.

The thickness of a cell in the said measurement was 10 mm in all cases.

## BEST MODE FOR PRACTICING THE INVENTION

Example 1: Quantitative analysis of $H_2O_2$ in the presence of a coloring-interference inhibitor:

(1) Preparation of reagents:

① Color reagent:

A color reagent was prepared such that 0.05 mmol/ℓ of BSdiproPM, 3,000 U/ℓ of POD, and 2% of a surface active agent or 0.2% of metal chelate as a coloring-interference inhibitor were contained in 0.05 M of phosphate buffer solution (pH 7.0).

②Albumin solution:

To 5 g of a human albumin (Albumin manufactured by Sigma Co., Ltd. Human Fraction V 96 - 99%) sample was added water to a total volume of 100 ml.

③ Uric acid standard solution:

A 150 ml/ℓ aqueous solution of uric acid was prepared according to the ordinary method.

④ $H_2O_2$ standard solutions:

Aqueous solutions containing 30 ppm and 60 ppm respectively were prepared as $H_2O_2$ standard solutions I and II.

(2) Measuring steps:

As a sample, a mixture of 50 µl of each of water, the albumin solution and the uric acid standard solution was used, and 3 mℓ of the color reagent were added to and mixed with the sample. Thereafter, 20 µℓ of the $H_2O_2$ standard solution I or II were added thereto, and the final mixture was incubated at 37° C for 10 minutes. Then, absorbance at a wavelength of 620 nm was measured with reference to a reagent blank as control.

Results are shown in Table 1.

Table 1

| Coloring-interference inhibitor \ $H_2O_2$ standard solution \ Sample | Water | | Albumin solution | | Uric acid standard solution | |
|---|---|---|---|---|---|---|
| | I | II | I | II | I | II |
| No addition | 0.684 | 1.372 | 0.624 | 1.248 | 0.094 | 0.142 |
| 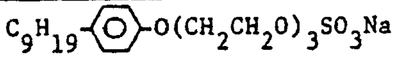 (anionic surface active agent) | 0.688 | 1.368 | 0.686 | 1.370 | 0.020 | 0.198 |
| Fe(III)-EDTA | 0.673 | 1.356 | 0.670 | 1.344 | 0.212 | 0.352 |
| Mn(II)-EDTA | 0.686 | 1.376 | 0.680 | 1.371 | 0.116 | 0.123 |
| Ni(II)-EDTA | 0.688 | 1.379 | 0.682 | 1.366 | 0.095 | 0.119 |

As is apparent from Table 1, the coloring interferences of albumin can be avoided by using the anionic surface active agent or the metal chelate according to the present invention. However, the interference of uric acid can not be avoided by these additives.

Example 2: Quantitative analysis of $H_2O_2$ in the presence of no coloring-interference inhibitor:

(1) Preparation of reagents:

① Color reagent:

Same as in Example 1 (no coloring-interference inhibitor contained)

② Uric acid standard solutions:

Aqueous solutions of uric acid at concentrations of 0, 25, 50, 75 and 100 mg/ℓ respectively were prepared according to the ordinary method.

③ $H_2O_2$ standard solutions:

Aqueous solutions containing 15, 30, 45 and 60 ppm of $H_2O_2$ respectively were prepared.

(2) Measuring steps:

Absorbances were measured by using the uric acid standard solutions as sample according to the measuring steps in Example 7.

Results are shown in Table 2 and in Fig.1 .

Table 2

| Concentration of uric acid standard solution (mg/ℓ) \ $H_2O_2$ standard solution (ppm) | 15 | 30 | 45 | 60 |
|---|---|---|---|---|
| 0 | 0.343 | 0.675 | 1.025 | 1.374 |
| 25. | 0.159 | 0.537 | 0.917 | 1.281 |
| 50 | 0.062 | 0.393 | 0.789 | 1.197 |
| 75 | 0.033 | 0.193 | 0.651 | 0.972 |
| 100 | 0.025 | 0.105 | 0.520 | 0.892 |

As can be seen from Table 2 and Fig. 1, negative errors are produced by the presence of uric acid, and the calibration curve is curved and does not pass through the origin. The negative error becomes larger with the increase in the concentration of uric acid, but is not proportional thereto.

Example 3: Quantitative analysis of $H_2O_2$ in the presence of coloring-interference inhibitor

(1) Preparation of reagents:

① Color reagent:

Same as in Example 1 (no coloring-interference inhibitor contained)

② Uric acid standard solutions:

Same as in the above Example 2

③ $H_2O_2$ standard solutions:

Same as in Example 2

(2) Measuring steps:

To the color reagent was added uricase to give a concentration of 200 U/ℓ, and absorbances were measured by using the resulting solution as in the case of Example 2.

Results are shown in Table 3.

Table 3

| $H_2O_2$ standard solution (ppm) / Concentration of uric acid (mg/ℓ) | 15 | 30 | 45 | 60 |
|---|---|---|---|---|
| 0 | 0.343 | 0.675 | 1.025 | 1.374 |
| 25 | 0.343 | 0.680 | 1.031 | 1.372 |
| 50 | 0.341 | 0.683 | 1.022 | 1.364 |
| 75 | 0.345 | 0.693 | 1.037 | 1.377 |
| 100 | 0.342 | 0.681 | 1.024 | 1.371 |

As seen from Table 3, the influences of uric acid are completely removed by using uricase, but no influence upon the measured value of $H_2O_2$ are produced thereby.

Example 4: Measurement of activity of serum monoamine oxidase

(1) Preparation of reagents:

① Substrate color reagent:

Allylamine, uricase, BSdiproPM, Emal®NC (Kao Soap Co., Ltd., trademark), and POD were dissolved in 20 mM phosphate buffer solution (pH 7.0) to give the concentrations of 15 m mol/ℓ, 200 U/ℓ, 0.03 m mol/ℓ and 5% respectively.

②Reaction terminator:

A 8.9 m mol/ℓ aqueous solution of sodium diethyldithiocarbamate was prepared.

③ Monoamine oxidase solutions:

5 IU/ℓ, 10 IU/ℓ and 20 IU/ℓ aqueous solutions were prepared by using bovine monoamine oxidase manufactured by Sigma Co., Ltd.

(2) Measuring steps:

50 μℓ of serum were sampled, and 3 mℓ of the substrate color reagent were added thereto. After incubation at 37°C for 30 minutes, 50 μℓ of the reaction terminator were added to and mixed with the solution. Then, absorbances at a wavelength of 620 nm were measured with reference to a reagent blank as control.

The absorbances were measured by using the monoamine oxidase solution as in the case of the serum, and a calibration curve was drawn.

Fig. 2 shows the calibration curve thus obtained.

The monoamine oxidase activity was determined from the calibration curve.

Example 5: Quantitative analysis of an extremely small amount of hemoglobin in serum in the presence of a coloring-interference inhibitor.

(1) Preparation of reagents:

① Reagent solution I:

2 g of leucomalachite green were dissolved in 100 mℓ of a mixed solvent of acetic acid and water at the volume ratio of 3:1.

② Reagent solution II:

30 g of glycine and 200 g of urea were dissolved in about 900 mℓ of water, and the solution was adjusted to pH 4.5 with hydrochloric acid, which was diluted with water to a total volume of 1,000 ml after the addition of 30 g of Emal®NC.

③ Reagent solution III:

Water was added to 1 mℓ of 30% $H_2O_2$ to a total volume of 100 ml.

④ Uricase solution:

Uricase was dissolved in 0.05 M phosphate buffer solution (pH 7.0) to give a concentration of 200 U/ℓ.

⑤ Serum:

Serum samples containing hemoglobin at the concentrations of 0.34, 0.68, 1.0, 1.5 and 2.0 mg/ℓ respectively were prepared by adding hemoglobin to a pool serum (containing 50 mg/ℓ of uric acid) containing no hemoglobin.

(2) Measuring steps:

To 0.1 mℓ of serum sampled were added 1 mℓ of the uricase solution and 11 mℓ of the reagent solution, which mixture was incubated at 37°C for 5 minutes. Then, 1 mℓ of the reagent solution II and 0.2 mℓ of the reagent solution III were added thereto, which mixture was further incubated at 37°C for 60 minutes. Then, absorbances at 620 mm were measured with reference to a reagent blank as control.

Fig.3 shows a calibration curve.

Example 6 : Quantitative analysis of an extremely small amount of hemoglobin in serum in the presence of a coloring-interference inhibitor

(1) Preparation of reagents:

① Reagent solution I:

Same as in Example 5

② Reagent solution II:

Same as the reagent solution II in Example 5 except that no Emal®NC was contained.

③ Reagent solution III:

Same as in Example 5

④ Serum:

Same as in Example 5

(2) Measuring steps:

To 0.1 mℓ of serum sampled were added 1 mℓ of 0.05 M phosphate buffer solution (pH 7.0), 1 mℓ of the reagent solution I, 1 mℓ of the reagent solution II and 0.2 mℓ of the reagent solution III, which mixture was subjected to the measurement of absorbances at 620 nm in the manner as in Example 5.

Example 7: Quantitative analysis of $H_2O_2$ in the presence of sodium azide

(1) Preparation of reagents:

① Color reagent:

A color reagent was prepared by dissolving BSdiproPM, peroxidase and sodium azide in 0.05 M phosphate buffer solution (pH 7.0) to give concentrations of 0.05 m mol/ℓ, 3,000 U/ℓ and 0.2% respectively.

② $H_2O_2$ standard solutions:

$H_2O_2$ aqueous solutions at concentrations of 15 mg/ℓ, 30 mg/ℓ, 45 mg/ℓ, 60 mg/ℓ and 75 mg/ℓ respectively were prepared.

③ Measuring steps:

To 20 μℓ of each of the $H_2O_2$ standard solutions were added 3 mℓ of the color reagent, and the mixture was incubated at 37°C for 10 minutes. Then, absorbances at 620 nm were measured with reference to a reagent blank as control.

Fig. 4 shows a calibration curve, which is a straight line passing through the origin. When the concentration of $H_2O_2$ is 15 mg/ℓ, the absorbance is 16.5% of the corresponding value in Example 8 in which no sodium azide was used.

Example 8: Quantitative analysis of $H_2O_2$ in the presence of no sodium azide

(1) Preparation of reagents:

① Color reagent:

A color reagent of the same composition at that in Example 6 except that no sodium azide was contained was prepared.

② $H_2O_2$ standard solutions:

Same as Example 7

(2) Measuring steps:

Absorbances were measured in the same manner as in Example 7.
Fig. 4 shows a calibration curve.

Example 9: Quantitative analysis of whole cholesterol in the presence of sodium azide:

(1) Preparation of reagents:

① Color reagent:

A color reagent was prepared by dissolving BSdiproPM, uricase, cholesterol esterase, cholesterol oxidase, peroxidase, sodium azide and Triton X-100 in 0.1 M tris-hydrochloric acid buffer solution (pH 7.0) to give concentrations of 0.05 m mol/ℓ 300 U/ℓ, 1,000 U/ℓ, cholesterol oxidase 150 U/ℓ, 3,000 U/ℓ, 0.2%, and 0.15% respectively.

② Cholesterol standard solutions:

Isopropanol solutions of cholesterol at concentrations of 100 mg/dℓ, 200 mg/dℓ, 300 mg/dℓ, 400 mg/dℓ and 500 mg/dℓ respectively were prepared.

(2) Measuring steps:

3 mℓ of the above coloring test solution were added to 20 μℓ of each of the cholesterol standard solutions and the mixture was incubated at 37°C for 10 minutes. Then, absorbances at 620 nm were measured with reference to a reagent blank as control, and a calibration curve was drawn.

Fig. 5 shows the calibration curve thus obtained, which is a straight line passing through the origin. In the calibration curve, the absorbance is 0.290 at the cholesterol concentration of 200 mg/dℓ, and this measurements is suitable for the measurement by using the ordinary photometer.

Example 10: Quantitative analysis of whole cholesterol in the presence of no sodium azide:

(1) Preparation of reagents:

① Color test solution:

A coloring test solution of the same composition of that in Example 9 except that no sodium azide was added was prepared.

② Cholesterol standard solution:

Same as in Example 9

(2) Measuring steps:

Absorbances were measured similarly to in Example 9. A calibration curve is shown in Fig. 5 . Although the calibration curve is a straight line passing through the origin , the absorbance has a high value of 0.731 at the cholesterol concentration of 200 mg/dℓ, and therefore accuracy is poor in the case of the ordinary photometer using a logarithm scale.

Example 11: Quantitative analysis $H_2O_2$ in the presence of β-CD

(1) Preparation of reagents:

① Color reagent:

β-CD and peroxidase were dissolved in 0.1 M tris-hydrochloric acid buffer solution pH 7.5 to give concentrations of 0.2% and 6,000 U/ℓ respectively. In this solution BSPM was dissolved at the concentration of 0.1 mM.

② $H_2O_2$ standard solutions:

$H_2O_2$ standard solutions at concentrations of 15 mg/ℓ, 30 mg/ℓ, 45 mg/ℓ, 50 mg/ℓ and 75 mg/ℓ respectively were prepared.

(2) Measuring steps:

3 mℓ of the color reagent were added to 20 μℓ of each $H_2O_2$ standard solution, which was incubated at 37°C for 10 minutes. Then, absorbances at 630 nm were measured with reference to a reagent blank as control.

Fig. 6 shows a calibration curve in which the absorbance is assigned to the ordinate, and the concentration of $H_2O_2$ is assigned to the abscissa. The calibration curve is a straight line passing through the origin. The absorbance at 15 mℓ/ℓ of $H_2O_2$ is 37.4% of the corresponding value in Example 13 using BSPM itself without CD being included.

Example 12: Quantitative measurement of $H_2O_2$ in the presence of α-CD

(1) Preparation of reagents:

① Color reagent:

A color reagent of the same composition as in Example 11 was prepared except that 0.2% of β-CD was substituted by 1% of α-CD.

② $H_2O_2$ standard solution:

Same as in Example 11

(2) Measuring steps:

Absorbances were measured in the same manner as in Example 11.

Fig. 6 shows a calibration curve, which is a straight line passing through the origin. The absorbance at 15 mg/ℓ of $H_2O_2$ is 55.2% of the corresponding value in Reference Example 13.

Example 13: Quantitative measurement of $H_2O_2$ in the presence of no CD:

(1) Preparation of reagents:

① Color reagent:

Peroxidase and BSPM were added and dissolved under stirring in 0.1 M tris-hydrochloric acid buffer solution (pH 7.5) to give concentrations of 6,000 U/ℓ and 0.1 mM. At that time, since BSPM was not completely dissolved, an undissolved portion thereof was filtered off after 10 minute stirring, and the filtrate was used as a color reagent.

② $H_2O_2$ standard solutions:

Same as in Reference Example 11

(2) Measuring steps:

Absorbances were measured in the same manner as in Example 11. A calibration curve is shown in Fig.6. Since the solubility of BSPM is low, the coloring reagent was insufficient, so that the calibration curve did not become a straight line.

Example 14: Quantitative analysis of $H_2O_2$ in the presence of β-CD

(1) Preparation of reagents:

① Color reagent:

BSdiproPM, β-CD and peroxidase were added to and dissolved in 0.1 M tris-hydrochloric acid buffer solution (pH 7.5) to give concentrations of 0.1 mM, 0.2% and 6,000 U/ℓ respectively.

② $H_2O_2$ standard solutions:

$H_2O_2$ standard solutions at concentrations of 15 mg/l, 30 mg/l, 45 mg/l, 50mg/l and 75 mg/l respectively, were prepared.

(2) Measuring steps:

Absorbances at a wavelength of 620 nm were measured by adding 3 ml of the color reagent to 20 µl of each $H_2O_2$ standard solution and incubating the mixture at 37°C for 10 minutes.

Then, absorbances at 630 nm were measured with reference to a reagent blank as control. Fig.7 shows

a calibration curve, which is a straight line passing through the origin. The absorbance at 15 mg/ℓ of $H_2O_2$ is 29.0% of the corresponding value in Example 16 in which BSdiproPM was used as it was without β-CD being included.

Example 15: Quantitative analysis of $H_2O_2$ in the presence of α-CD

(1) Preparation of reagents:

① Color reagent:

A color reagent of the same composition as in Example 14 was prepared except that 0.2% of β-CD was substituted by 1% of α-CD.

② $H_2O_2$ standard solutions:

Same as in Example 11.

(2) Measuring steps:

Absorbances were measured in the same manner as in Example 14. Fig. 7 shows a calibration curve which is a straight line passing through the origin. The absorbance at 15 mg/ℓ of $H_2O_2$ is 38.7% of the corresponding value in Example 16.

Example 16: Quantitative analysis of $H_2O_2$ in the presence of no CD:

(1) Preparation of reagents:

① Color reagent:

A color reagent of the same composition as in Example 14 was prepared except that no β-CD was added.

② $H_2O_2$ standard solutions:

Same as in Example 14

(2) Measuring steps:

Absorbances were measured in the same manner as in Example 14. Fig.7 shows a calibration curve which is a straight line passing through the origin.

Example 17: Quantitative analysis of $H_2O_2$ in the presence of β-CD

(1) Preparation of reagents:

① Color reagent:

BSproPM, β-CD and peroxidase were added to and dissolved in 0.1 M tris-hydrochloric acid buffer solution (pH 7.5) to give concentrations of 0.1 mM, 0.2% and 6,000 U/ℓ respectively.

② $H_2O_2$ standard solutions:

Same as in Example 11

(2) Measuring steps:

Absorbances were measured in the same manner as in Example 11. Fig. 8 shows a calibration curve, which is a straight line passing through the origin. The absorbance at 15 mg/ℓ of $H_2O_2$ is 38.8% of the corresponding value in Example 19 in which BSproPM was used as it was without β-CD being included.

Example 18: Quantitative analysis of $H_2O_2$ in the presence of α-CD

(1) Preparation of reagents:

① Color reagent:

A color reagent of the same composition as in Example 17 was prepared except that 0.2% of β-CD was substituted by 1% of α-CD.

② $H_2O_2$ standard solutions:

Same as in Example 11

(2) Measuring steps:

Absorbances were measured in the same manner as in Example 11. Fig. 8 shows a calibration curve, which is a straight line passing through the origin. The absorbance at 15 mg/ℓ of $H_2O_2$ is 53.7% of the corresponding value in Example 19.

Example 19: Quantitative analysis of $H_2O_2$ in the presence of no CD:

(1) Preparation of reagents:

① Color reagent:

A color reagent of the same composition as in Example 17 was prepared except that no β-CD was added.

② $H_2O_2$ standard solutions:

Same as in Example 11

(2) Measuring steps:

Absorbances were measured in the same manner as in Example 11. Fig.8 shows a calibration curve, which is a straight line passing through the origin.

Example 20: Quantitative analysis of total cholesterol in the presence of β-CD

(1) Preparation of reagents:

① Color reagent:

BSdiproPM, uricase, cholesterol oxidase, peroxidase,β-CD and Triton X-100 were added to and dissolved in 0.1 M tris-hydrochloric acid buffer solution (pH 7.0) to give concentrations of 0.05 mM, 300 U/ℓ, 150 U/ℓ, 3,000 U/ℓ, 0.2% and 0.15% respectively to obtain a color reagent.

② Cholesterol standard solutions:

Isopropanol solutions of cholesterol at concentrations of 100 mg/dℓ, 200 mg/dℓ, 300 mg/dℓ, 400 mg/dℓ and 500 mg/dℓ respectively were prepared.

(2) Measuring steps:

To 20 μℓ of each of the cholesterol standard solutions of the above respective concentrations were added 3 mℓ of the coloring test solution and the mixture was incubated at 37°C for 10 minutes. Then, absorbances at a wavelength of 620 nm were measured with reference to a blank reagent as control, and a calibration curve was drawn. Fig.9 shows the calibration curve thus obtained, which is a straight line passing through the origin. The absorbance at the cholesterol concentration of 200 mg/dℓ is 0.210, which absorbance is suitable for the measurement by using the ordinary photometer.

Example 21: Quantitative analysis of total cholesterol in the presence of modified β-CD

(1) Preparation of reagent:

① Color reagent:

A color reagent was prepared by dissolving BSdiproPM, uricase, cholesterol oxidase, peroxidase, heptakis(2,6-di-O-methyl)-β-cyclodextrin and Triton X-100 in 0.1 M tris-hydrochloric acid buffer solution (pH 7.0) to give concentrations of 0.05 mM, 300 U/ℓ, 150 U/ℓ, 3,000 U/ℓ, 0.3% and 0.15% respectively.

② Cholesterol standard solutions:

Same as in Example 20

(2) Measuring steps:

According to the measuring steps in Example 20, absorbances at a wavelength of 620 nm were measured, and a calibration curve was drawn. Fig. 10 shows the calibration curve thus obtained, which is a straight line passing through the origin. The absorbance at the cholesterol concentration of 200 mg/dℓ is

0.220, which absorbance is suitable for the measurement by using the ordinary photometer.

Example 22: Quantitative analysis of total cholesterol in the presence of no β-CD

(1) Preparation of reagents:

① Color reagent:

A color reagent of the same composition as in Example 20 was prepared except that no β-CD was added.

② Cholesterol standard solutions:

Same as in Example 20

(2) Measurement steps:

Absorbances were measured in the same manner as in Example 20. A calibration curve is shown in Fig.9 (Fig. 10). Although the calibration curve is a straight line passing through the origin, the absorbance at the cholesterol concentration of 200 mg/dℓ has a high value of 0.731. Thus, the accuracy in the measurement by using a photometer using a logarithm scale is poor.

Example 23: Quantitative analysis of triglyceride in the presence of β-CD

(1) Preparation of reagents:

① Color reagent:

A solution in 0.05 M tris-hydrochloric acid buffer solution (pH 7.5) was prepared containing 0.05 mM of BSdiproPM, 300 U/ℓ of uricase 5,000 U/ℓ of lipoprotein lipase, 4,000 U/ℓ of glycerokinase, 1,000 U/ℓ of glycerol-3-phosphoric acid oxidase, 3,000 U/ℓ of peroxidase, 0.2% of β-CD, 1,000 mg/ℓ of ATP, 5 mM of magnesium chloride, 0.1% of Triton X-405 and 1.0% of Emal®NC (Kao Soap Co., Ltd., trademark)

② Glycerol standard solutions:

Aqueous glycerol solutions at concentrations of 10.4 mg/dℓ, 20.8 mg/dℓ, 31.2 mg/dℓ, 41.6 mg/dℓ, 52.0 mg/dℓ and 62.4 mg/dℓ respectively (corresponding to the concentration of 100 mg/dℓ, 200 mg/dℓ, 300 mg/dℓ, 400 mg/dℓ, 500 mg/dℓ, and 600 mg/dℓ as triolein respectively)were prepared.

(2) Measuring steps:

3 mℓ of the color reagent were added to 20 μℓ of sampled serum, which mixture was incubated at 37°C for 10 minutes. Then, absorbances at a wavelength of 620 nm were measured with reference to a reagent blank as control.

Furthermore , 3 mℓ of the color reagent were added to 20 μℓ of each of the sampled glycerol standard solution of the above respective concentrations, which mixtures were subjected to the measurement of absorbances in the same manner as in the case of the serum to obtain a calibration curve. Fig. 11 shows the calibration curve thus obtained, which is a straight line passing through the origin. The absorbance at

the triglyceride concentration of 200 mg/dℓ is 0.185, which is suitable for the measurement by using the ordinary photometer.

Example 24: Quantitative analysis of triglyceride in the presence of no $\beta$-CD

(1) Preparation of reagents:

① Color reagent:

A color reagent of the same composition in Example 23 was prepared except that no$\beta$-CD was added.

② Glycerol standard solutions:

As as in Example 23

(2) Measuring steps:

Similarly to Example 23, the glycerol standard solutions of the above respective concentrations were colored, and their absorbances were measured, from which a calibration curve was drawn. The calibration curve thus drawn is shown in Fig. 11. Although the calibration curve is a straight line passing through the origin, the absorbance at the triglyceride concentration of 300 mg/dℓ has a high value of 0.951. Therefore, the accuracy in the measurement by using a photometer of a logarithm scale is poor.

In the following Table is shown a comparison in the measured values of serum triglyceride between Example 23 and Example 24.

Table 4

| Comparison in the measured value of serum triglyceride | | | | | |
|---|---|---|---|---|---|
| Serum | Example 23 | Example 24 | Serum | Example 23 | Example 24 |
| 1 | 83 mg/dℓ | 78 mg/dℓ | 7 | 267 mg/dℓ | 265 mg/dℓ |
| 2 | 600 | 618 | 8 | 379 | 373 |
| 3 | 96 | 106 | 9 | 187 | 193 |
| 4 | 77 | 68 | 10 | 98 | 101 |
| 5 | 133 | 134 | | | |
| 6 | 115 | 119 | Average | 203.5 | 205.5 |

$\gamma$ = 0.999

As shown in Table 4, the significant value between Examples 23 and 24 are $\gamma$ = 0.999, and they exhibit a good correlation.

## INDUSTRIAL APPLICABILITY

As mentioned above, the invention can effectively avoid the influences of the substances interfering the color development, the coloring method of the invention is suitable for the quantitative measurement of a small amount of the component in a sample from a living organism, particularly a body fluid such as blood

or urine. Further, since the coloring sensitivity can be appropriately adjusted according to the present invention, the use range at which the coloring reagent may be used according to the present invention is not limited to the quantitative measurement of a small amount of the component. Thus, the method can be applied to a wide range of uses such as clinically chemical analysis.

## Claims

1. A method of quantitatively measuring an oxidative substance by using as a coloring reagent a triphenyl methane type leuco coloring matter represented by the general formula (I):

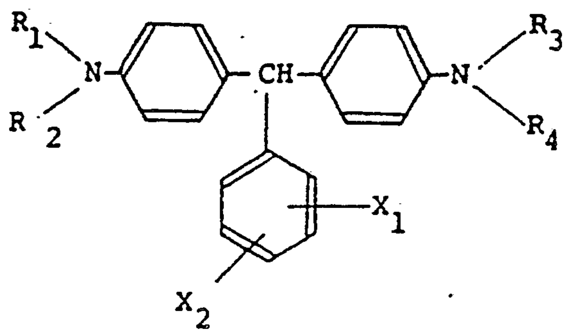

(I)

wherein $R_1$, $R_2$, $R_3$ and $R_4$, which may be the same or different from one another, represent a hydrogen atom or a $C_{1-3}$-alkyl group, and $X_1$ and $X_2$, which may be the same as or different from each other, represent a hydrogen atom, $-SO_3M_1$, $-COOM_2$, $-O(CH_2)_mSO_3M_3$, $-O(CH_2)_nCOOM_4$ or $-N(R_5)(R_6)$ in which $M_1$, $M_2$, $M_3$ and $M_4$ represent a hydrogen atom, an alkali metal ion or $NH_4^+$ , $R_5$ and $R_6$ independently represent a hydrogen atom or a lower alkyl group, and m and n represent an integer of 2 - 4, characterized by using at least one kind of (i) uricase, (ii) an anionic surface active agent and (iii) a metal chelate compound, whereby influences of a coloring-interfering substance is avoided.

2. The method of quantitatively measuring an oxidative substance according to Claim 1 , wherein the oxidative substance is hydrogen peroxide generated by acting an oxidation enzyme upon a substrate or a substance produced in an enzyme reaction.

3. The method of quantitatively measuring an oxidative substance according to Claim 2 , wherein the coloring reagent is oxidation-colored in the presence of peroxidase, and coloring is colorimetrically measured, whereby $H_2O_2$ is quantitatively measured.

4. The method of quantitatively measuring an oxidative substance according to Claim 2 , wherein a body fluid component selected from the group consisting of glucose, cholesterol, triglyceride, phospholipid, choline, creatine, creatinine, bile acid and monoamine oxidase is quantitatively measured by quantitatively measuring $H_2O_2$.

5. The method of quantitatively measuring an oxidative substance according to Claim 1 , wherein a peroxidase-like substance in a body fluid is quantitatively measured by quantitatively measuring $H_2O$.

6. The method of quantitatively measuring an oxidative substance according to Claim 5, wherein the peroxidase-like substance is one selected from hemoglobin and a hem compound other than hemoglobin.

7. The method of quantitatively measuring an oxidative substance according to any one of Claims 1 - 6 , wherein the coloring-interfering substance is uric acid, and the influences of the uric acid are avoided by using uricase.

8. The method of quantitatively measuring an oxidative substance according to any one of Claims 1 - 6 , wherein the coloring-interfering substance is protein, and the influences of the protein are avoided by using the anionic surface active agent.

9. The method of quantitatively measuring an oxidative substance according to any one of Claims 1 - 6 , wherein the coloring-interfering substance is protein, and the influences of protein are avoided by using the metal chelate compound.

10. The method of quantitatively measuring an oxidative substance according to any one of Claims 1 - 6 , wherein the coloring-interfering substance is uric acid and protein, and the influences of the uric acid are avoided by using uricase, while those of the protein are avoided by using the anionic surface active agent.

11. The method of quantitatively measuring an oxidative substance according to any one of Claims 1 - 6 , wherein the coloring-interfering substance is uric acid and protein, and the influences of the uric acid are

avoided by using uricase, while those of the protein are avoided by using the metal chelate compound.

12. The method of quantitatively measuring an oxidative substance according to Claim 8 which use the anionic surface active agent represented by one of the general formulae (II) and (III):

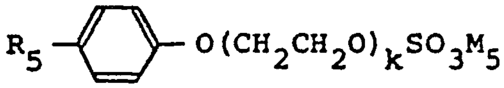 (II)

$$R_6O(CH_2CH_2O)_\ell SO_3M_6 \qquad (III)$$

in which $R_5$ is an alkyl group of 8 - 9 carbon atoms, $R_6$ represents an alkyl group of 8 - 18 carbon atoms, $M_5$ and $M_6$ each represent an alkali metal ion, an ammonium ion, or a quaternary ammonium ion, and k and $\ell$ each represent an integer of 1 - 6.

13. The method of quantitatively measuring an oxidative substance according to Claim 9 , wherein the metal chelate compound is a metal-EDTA (ethylenediamine tetraacetic acid) chelate.

14. A method of quantitatively measuring an oxidative substance by using as a coloring reagent a triphenyl methane type leuco coloring matter represented by the general formula (I):

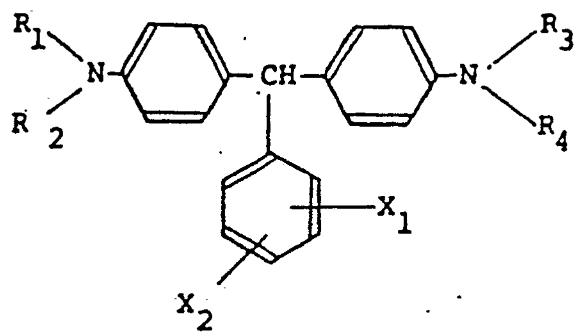 (I)

wherein $R_1$, $R_2$, $R_3$ and $R_4$, which may be the same or different from one another, represent a hydrogen atom or a $C_{1-3}$-alkyl group, and $X_1$ and $X_2$, which may be the same as or different from each other, represent a hydrogen atom, $-SO_3M_1$, $-COOM_2$, $-O(CH_2)_mSO_3M_3$, $-O(CH_2)_nCOOM_4$ or $-N(R_5)(R_6)$ in which $M_1$, $M_2$, $M_3$ and $M_4$ represent a hydrogen atom, an alkali metal ion or $NH_4^+$ , $R_5$ and $R_6$ independently represent a hydrogen atom or a lower alkyl group, and m and n represent an integer of 2 - 4, characterized in that an azide is used to adjust the coloring sensitivity.

15. The method of quantitatively measuring an oxidative substance according to Claim 14 , wherein the azide is sodium azide.

16. The method of quantitatively measuring an oxidative substance according to Claim 14 , wherein the coloring component is oxidation-colored in the presence of peroxidase, and coloring thereof is colorimetrically measured.

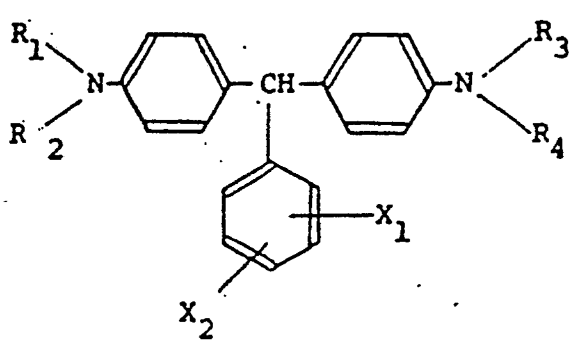 (I)

wherein $R_1$, $R_2$, $R_3$ and $R_4$, which may be the same or different from one another, represent a hydrogen atom or a $C_{1-3}$-alkyl group, and $X_1$ and $X_2$, which may be the same as or different from each other, represent a hydrogen atom, $-SO_3M_1$, $-COOM_2$, $-O(CH_2)_mSO_3M_3$, $-O(CH_2)_nCOOM_4$ or $-N(R_5)(R_6)$ in which

$M_1$, $M_2$, $M_3$ and $M_4$ represent a hydrogen atom, an alkali metal ion or $NH_4^+$, $R_5$ and $R_6$ independently represent a hydrogen atom or a lower alkyl group, and m and n represent an integer of 2 - 4, said triphenyl methane derivative being included with cyclodextrin or modified cyclodextrin.

21. The method of quantitatively measuring an oxidative substance according to Claim 20 , wherein the coloring component is oxidation-colored in the presence of peroxidase, and coloring thereof is colorimetrically measured.

22. The method of quantitatively measuring an oxidative substance according to Claim 20 , wherein the oxidative substance is hydrogen peroxide, and said hydrogen peroxide is generated by an enzyme reaction.

23. The method of quantitatively measuring an oxidative substance according to Claim 22, wherein the hydrogen peroxide is hydrogen peroxide which is generated by the enzyme reaction in a quantitative measurement of an extremely small amount of a component in a sample from a living organism.

24. The method of quantitatively measuring an oxidative substance according to Claim 23, wherein the quantitative measurement of the extremely small amount of the component in the sample from the living organism is a quantitative measurement of one selected from a substrate and an enzyme activity in a sample from a living organism, which is carried out by acting an oxidation enzyme upon a substrate or substance produced by enzyme reaction to produce hydrogen peroxide, and quantitatively measuring hydrogen peroxide thus produced.

FIG. 1

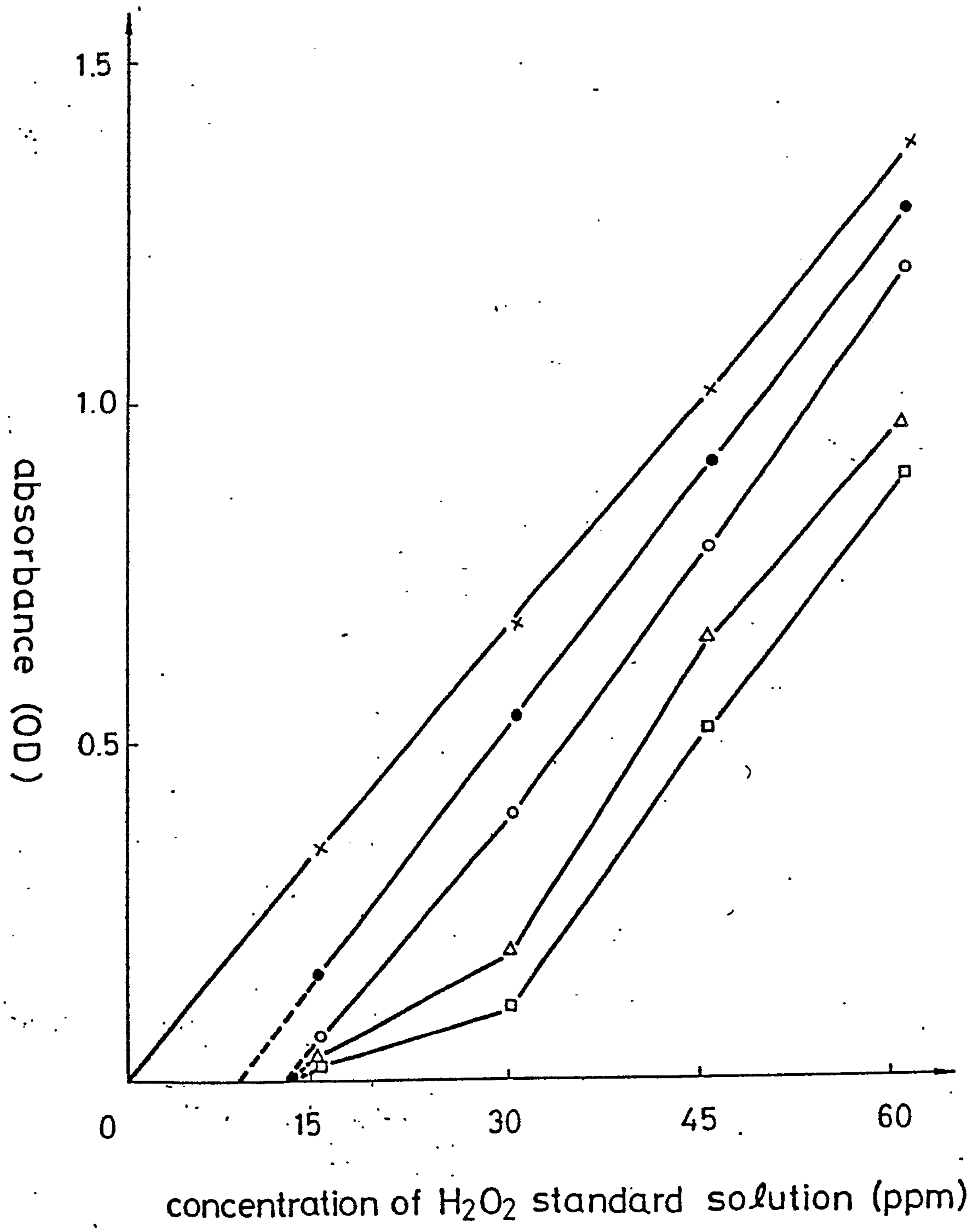
1.5
1.0
0.5
absorbance (OD)
0
15
30
45
60
concentration of $H_2O_2$ standard solution (ppm)

FIG. 2

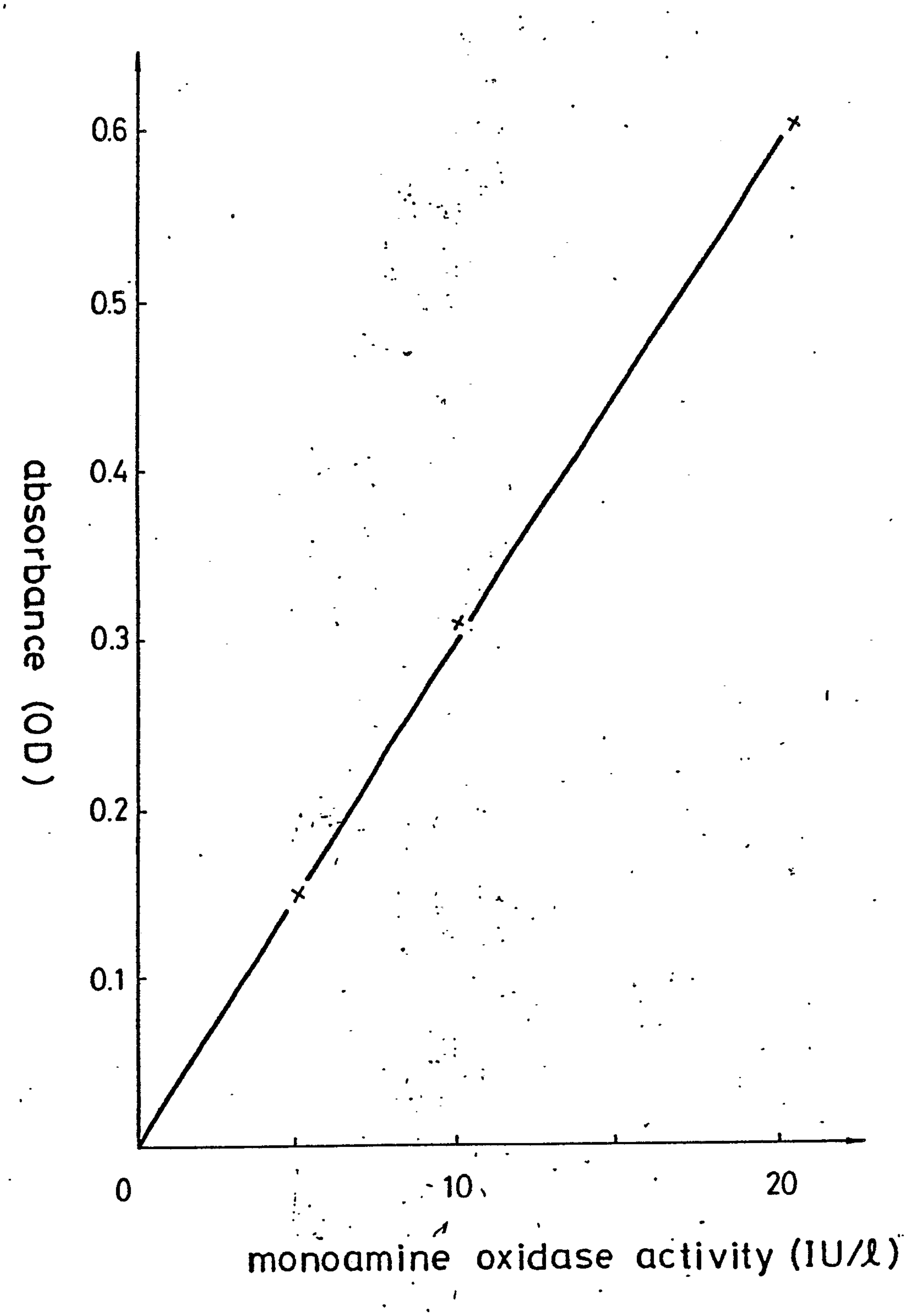
0.6
0.5
0.4
0.3
0.2
0.1
absorbance (OD)
0
10
20
monoamine oxidase activity (IU/ℓ)

FIG. 3

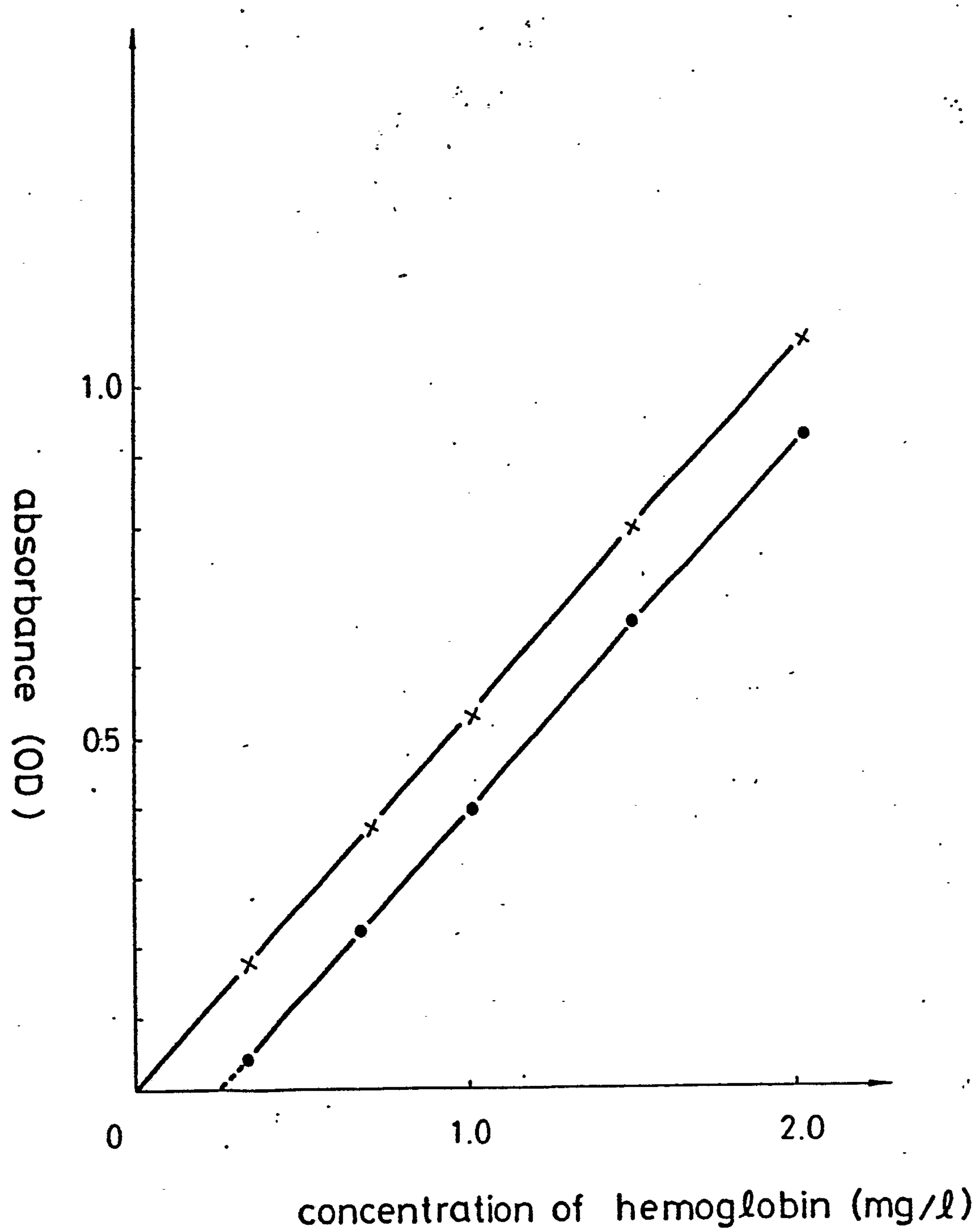

FIG. 4

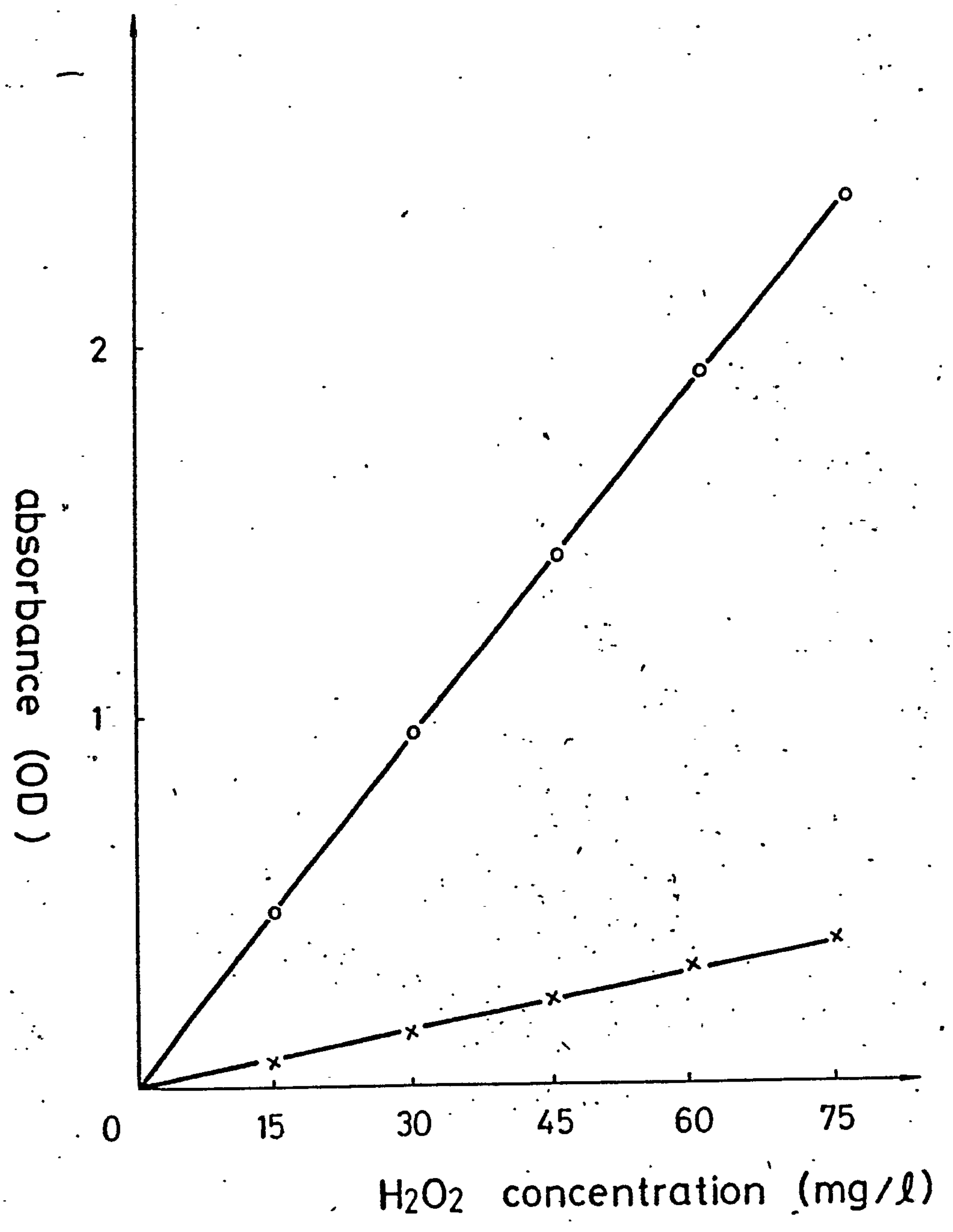

FIG.5

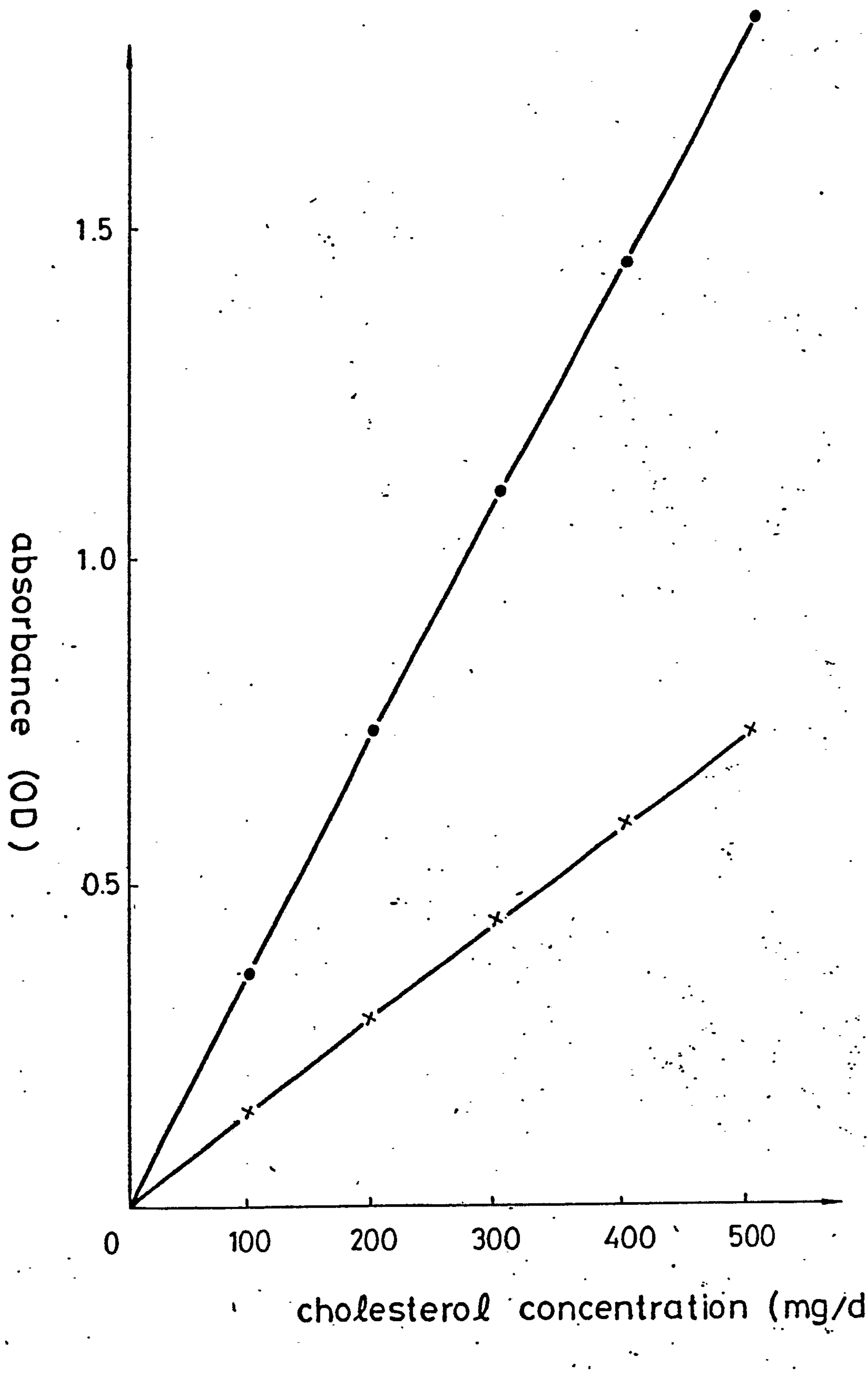

absorbance (OD)
1.5
1.0
0.5
0
100
200
300
400
500
cholesterol concentration (mg/dl)

FIG. 6

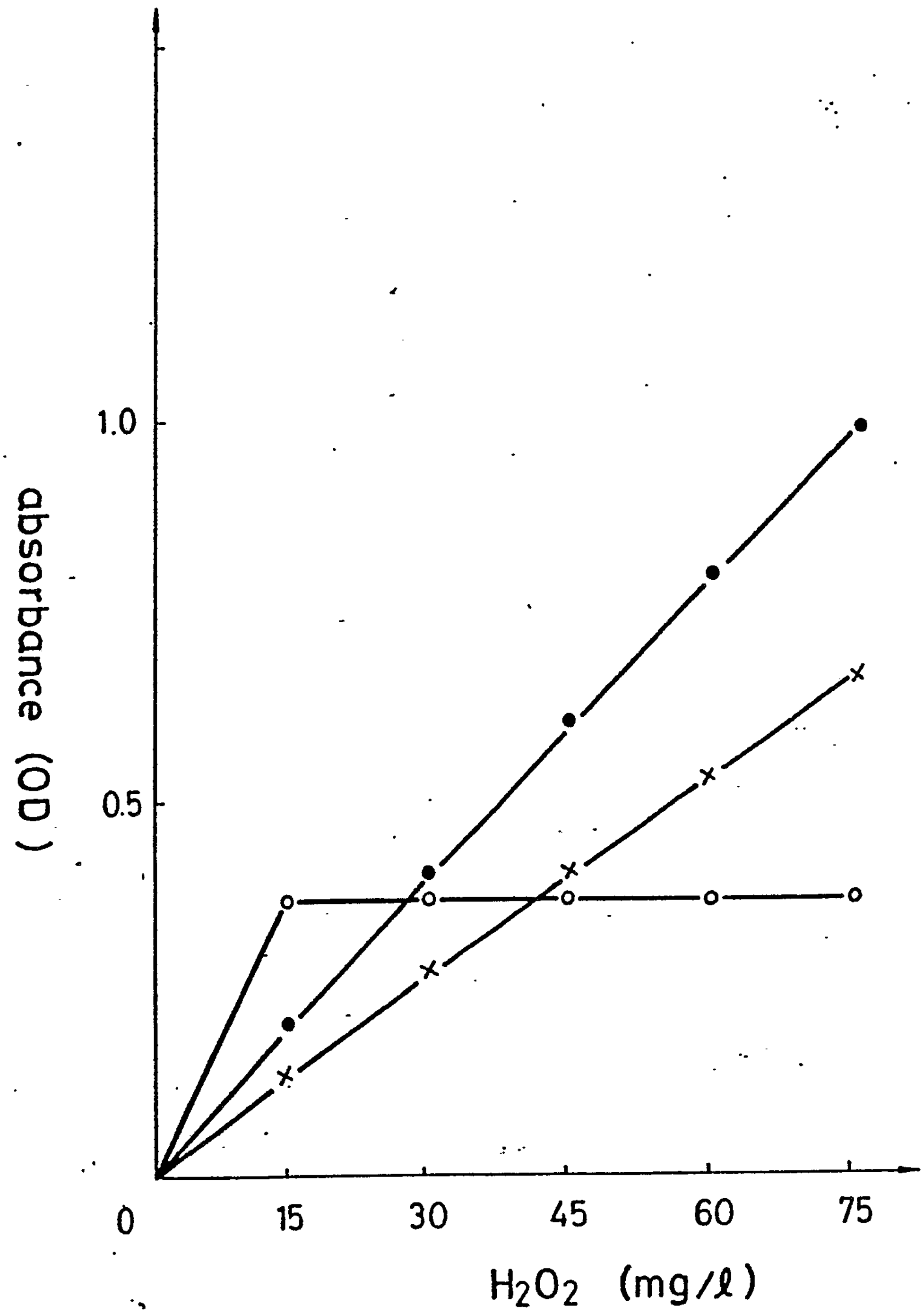

FIG. 7

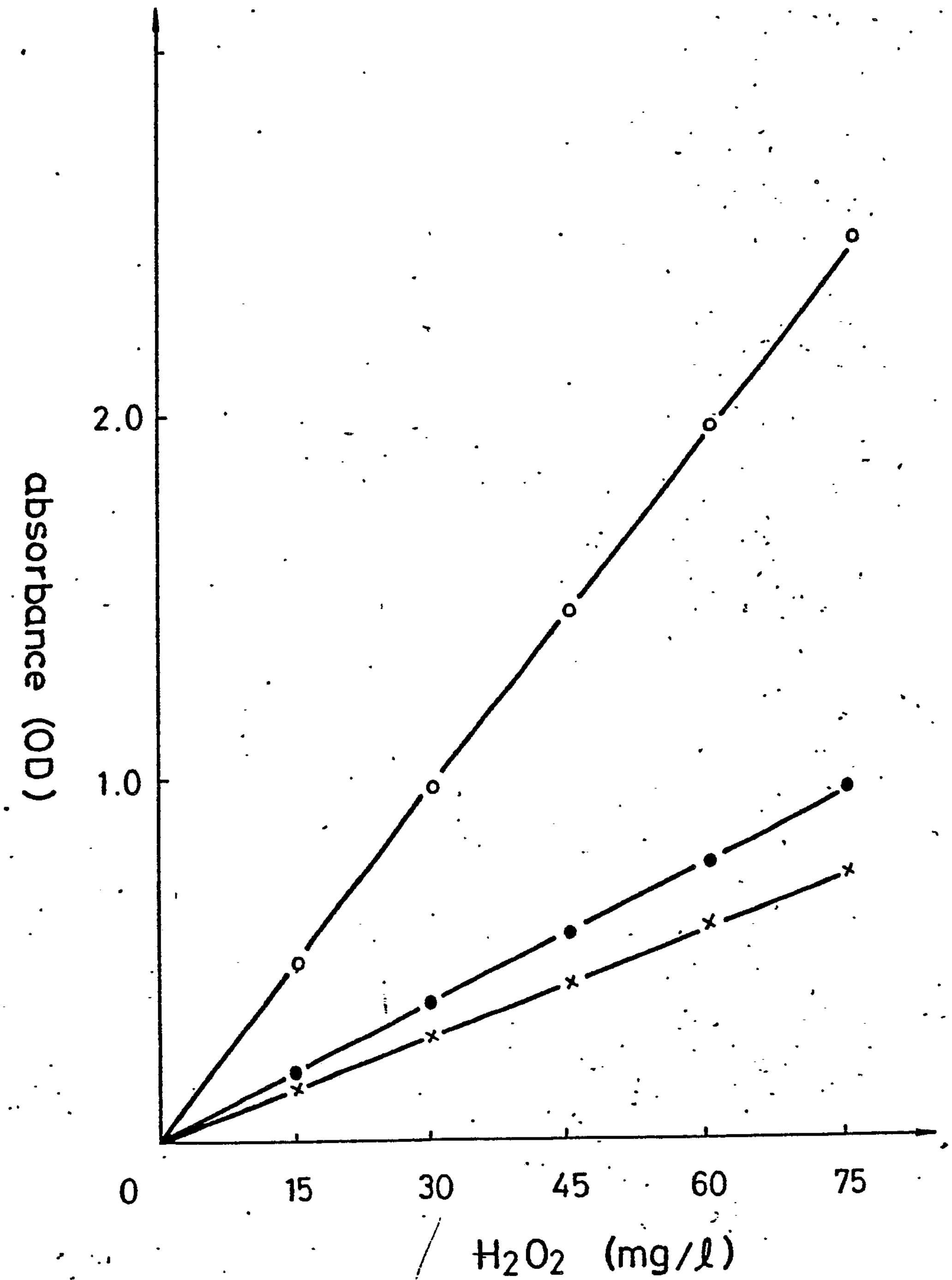

FIG. 8

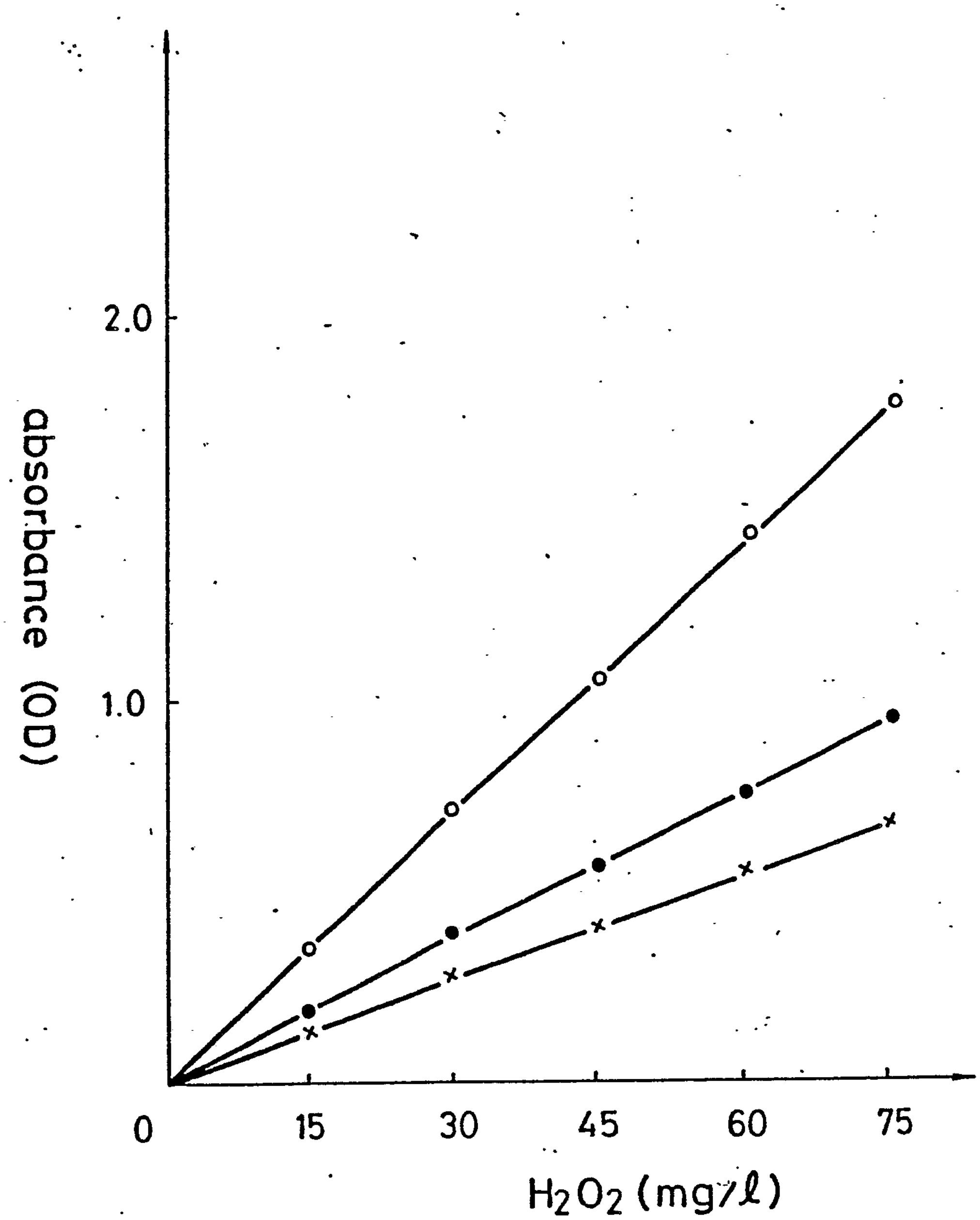

FIG. 9

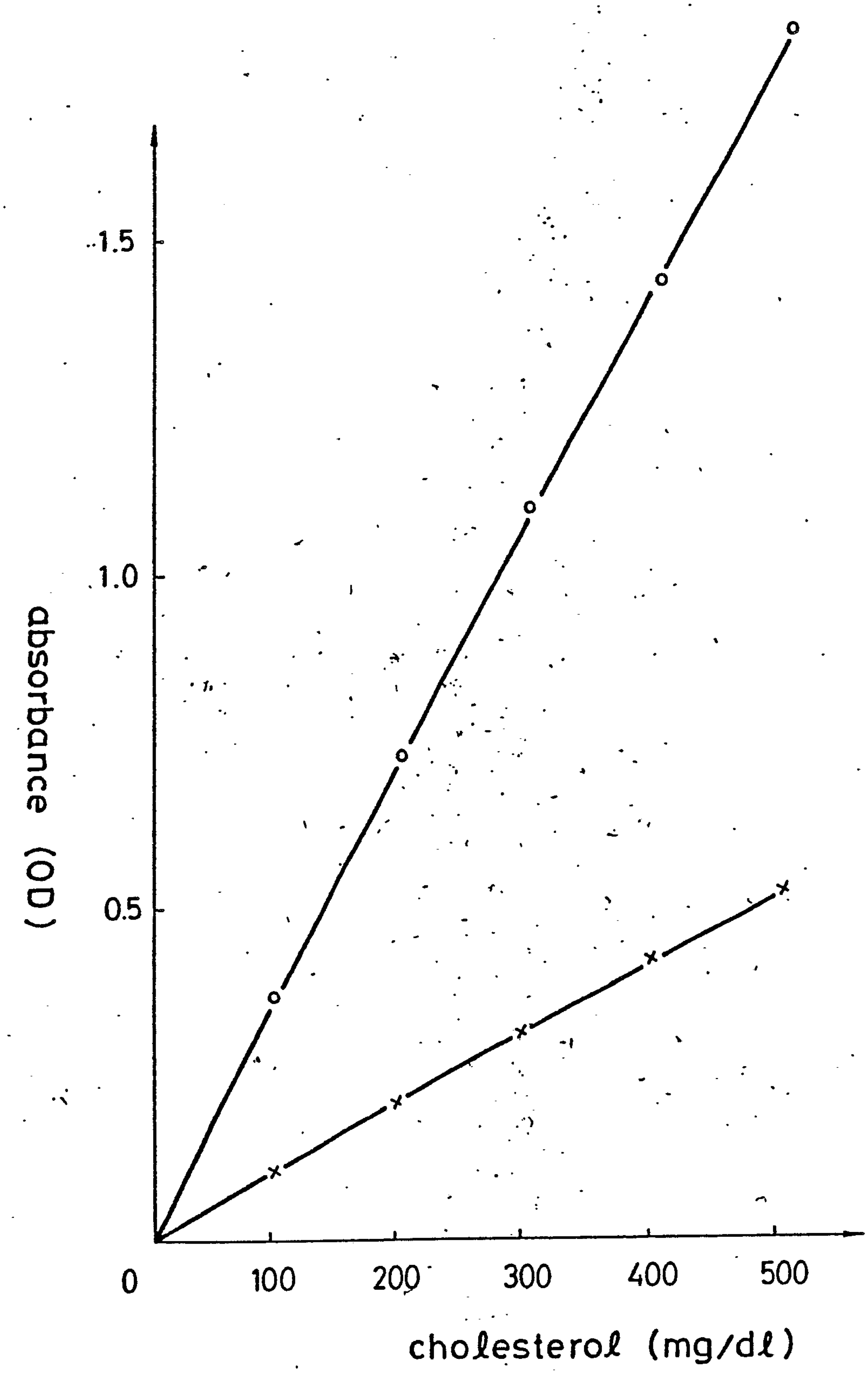

FIG.10

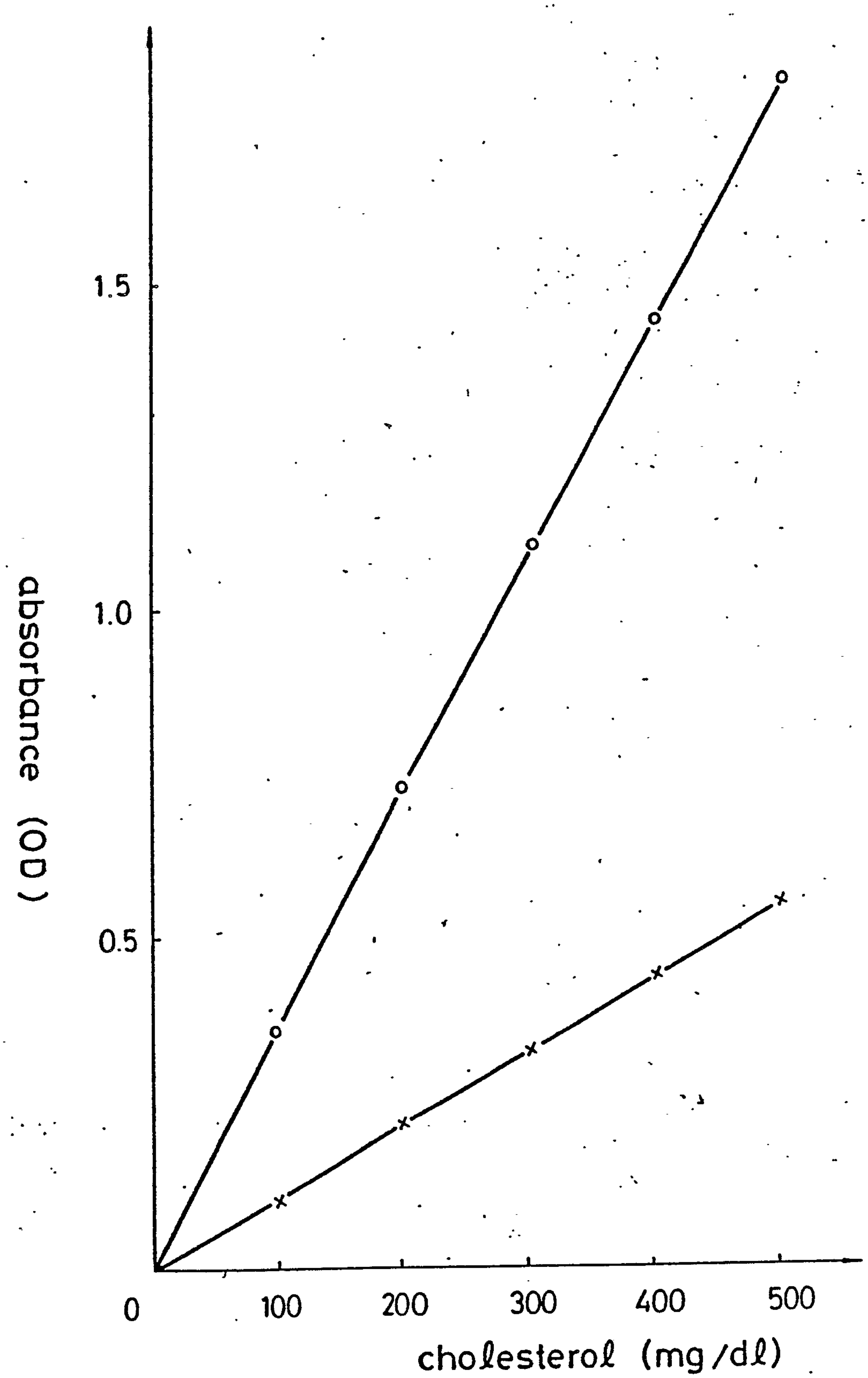

FIG.11

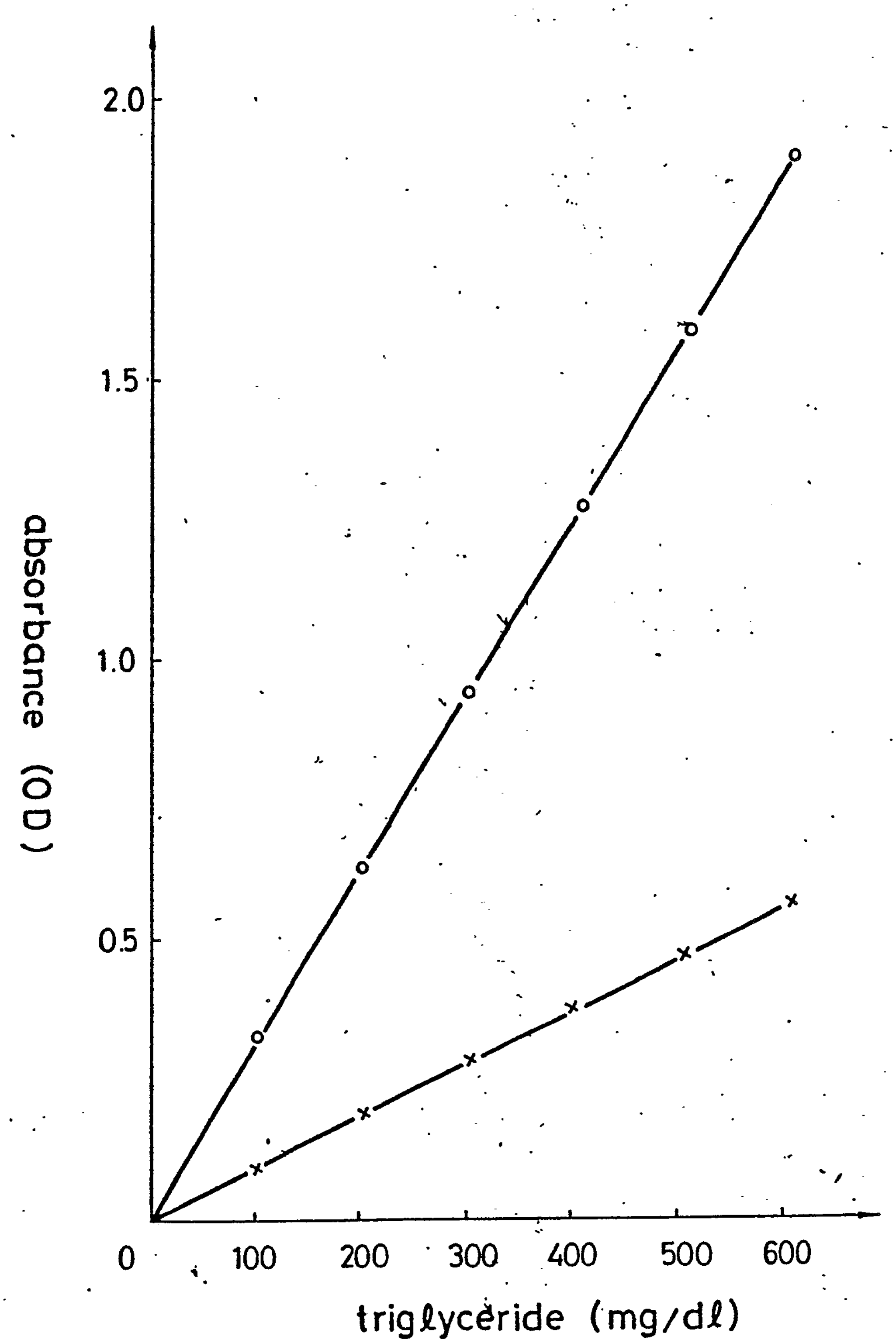
2.0
1.5
1.0
0.5
0
absorbance (OD)
100
200
300
400
500
600
triglyceride (mg/dℓ)